# FASCICULE DE BREVET EUROPEEN

(11) **EP 1 932 505 B1**
(45) Date de publication et mention de la délivrance du brevet: **26.12.2012**
(21) Numéro de dépôt: 07121456.3
(22) Date de dépôt: 23.11.2007
(51) Int. Cl.: A61K 8/06, A61K 8/81, A61K 8/892, A61Q 1/00, A61Q 19/00, A61Q 19/08, A61K 8/894

(54) **Emulsion e/h à effet correcteur pour la peau**
W/O-Emulsion mit einer hautkorrigierenden Wirkung
W/O emulsion with a skin-correcting effect

(30) Priorité: 11.12.2006 FR 0655421
(43) Date de publication de la demande: 18.06.2008
(73) Titulaire: L'Oréal, 75008 Paris (FR)
(72) Inventeur: Lorant, Raluca, 94320, THIAIS (FR)
(74) Mandataire: Duvert, Sandra

(56) Documents cités:
- EP-A- 1 530 961

## Description

L'invention se rapporte à une composition cosmétique se présentant sous forme d'une émulsion eau-dans-huile (E/H) comportant une grande quantité de phase aqueuse, et contenant un émulsionnant siliconé, un polymère hydrophile, et une charge à base de silicium.

L'invention a aussi pour objet, les utilisations de la dite composition, en particulier comme produit de soin de la peau et plus particulièrement pour atténuer les irrégularités visibles ou tactiles de la surface de la peau et notamment corriger les imperfections telles que les dyschromies, les taches et les rides, tout en hydratant la peau.

Le document D1 décrit des émulsions cosmétiques de préférence du type huile-dans-eau (H/E) contenant des portions de sphères creuses organosiliconées pour estomper les défauts de surface de la peau.

Dans le domaine cosmétique, les émulsions de type eau-dans-huile (E/H) sont reconnues comme étant efficaces pour le soin des peaux sèches du fait qu'elles comportent une phase continue huileuse et qu'elles permettent donc de former à la surface de la peau, un film lipidique qui prévient la perte d'eau transépidermique et protège la peau des agressions extérieures. Ces émulsions sont particulièrement appropriées pour protéger et nourrir la peau, et en particulier pour traiter les peaux sèches. Toutefois, il est souvent reproché aux émulsions E/H d'être trop grasses et collantes et de présenter une stabilité fragile. Elles n'apportent pas de fraîcheur et sont généralement trop riches en huiles pour être utilisées pendant l'été ou dans les pays chauds.

Pour surmonter ces inconvénients il a été proposé de préparer des émulsions E/H à forte teneur en eau. Ainsi, le document EP-A-1,068,851 décrit l'utilisation d'un organopolysiloxane élastomère oxyalkyléné comme émulsionnant pour stabiliser des émulsions E/H contenant au moins 70% de phase aqueuse. Toutefois, les compositions obtenues selon ce document, bien qu'apportant effectivement une certaine fraîcheur à l'application grâce au taux de phase aqueuse très important, présentent l'inconvénient de manquer de confort et de ne pas apporter suffisamment d'effets de soin sur la peau, tels que la nutrition de la peau, et des effets visibles et ressentis, en particulier du fait que la phase huileuse comporte essentiellement des huiles volatiles qui, donc, ne restent pas sur la peau et ne peuvent nourrir la peau.

Par ailleurs, le document EP-A-1,136,058 propose également des émulsions E/H apportant de la fraîcheur à l'application sur la peau, grâce à la présence d'un taux élevé de phase aqueuse. Toutefois, comme dans le document précédemment cité, ces compositions stabilisées par un organopolysiloxane élastomère oxyalkyléné posent les mêmes limites :dans leur apport bénéfique sur la peau, à savoir un manque de confort et d'effets nourrissants pour la peau.

Par ailleurs, quand on veut introduire des charges dans des compositions telles que décrites dans ces documents, il est souvent nécessaire d'utiliser une quantité plus importante de tensioactif émulsionnant. De plus, l'ajout de charges peut renforcer l'impression de manque de confort de la composition sur la peau voire même donner un toucher rêche. Or, l'addition de charges est particulièrement recherchée, par exemple pour améliorer le toucher et donner un effet matifiant sur la peau ou camoufler les imperfections de la peau.

Il subsiste donc le besoin d'une composition sous forme d'émulsion E/H qui, tout en donnant un effet de fraîcheur à l'application grâce à la présence d'un taux élevé de phase aqueuse, ne présente pas les inconvénients de l'art antérieur et notamment qui soit douce à l'application, qui procure :des effets cosmétiques immédiats tels que la matité et la diminution des imperfections de la peau, tout en apportant de l'hydratation, et qui soient stables en présence de charges.

La demanderesse a maintenant trouvé de manière surprenante que l'on pouvait obtenir une composition de ce type en utilisant une émulsion eau dans huile contenant un émulsionnant siliconé, un polymère hydrophile, et comme charges, des charges spécifiques à base de silicium.

L'invention a pour objet une composition pour application topique sous forme d'émulsion eau-dans-huile, comprenant une phase aqueuse dispersée dans une phase huileuse, et contenant (1) au moins un émulsionnant siliconé, (2) au moins un polymère hydrophile choisi parmi les polymères acryliques réticulés et les polymères d'acide 2-acrylamido 2-méthylpropane sulfonique, et (3) au moins une charge à base de silicium, la quantité de phase aqueuse étant d'au moins 60 % en poids par rapport au poids total de la composition.

On entend par «quantité de phase aqueuse » la quantité de l'ensemble de la phase aqueuse, c'est-à-dire l'eau et touts les composés hydrosolubles.

La composition étant pour application topique, elle contient un milieu physiologiquement acceptable. On entend par « milieu physiologiquement acceptable » dans la présente invention, un milieu non toxique, compatible avec la peau (y compris l'intérieur des paupières), les muqueuses, les cheveux ou les lèvres d'êtres humains. La composition constituant de préférence une composition cosmétique, elle a un aspect, une odeur et un toucher agréables.

Les compositions de l'invention présentent l'avantage de donner un effet de fraîcheur lors de l'application sur la peau, tout en ayant une bonne efficacité d'hydratation de la peau et tout en montrant une bonne stabilité dans le temps, et tout en apportant un grand agrément cosmétique car elles sont très confortables lors de l'application sur la peau (pas de tiraillement, douceur). Même quand elles sont épaisses, ces compositions deviennent fluides et fraîches sur la peau. Les effets immédiats ressentis et/ou mesurés sont l'hydratation, le confort, ainsi que des effets optiques excellents tels que l'effet matifiant, le camouflage des imperfections de la peau (taches, ridules), et l'effet éclaircissant immédiat.

### Emulsionnant siliconé

On entend par « émulsionnant siliconé », un composé siliconé contenant une chaîne hydrophile et capable d'émulsionner une phase aqueuse dans une phase huileuse. Cet émulsionnant siliconé peut être choisi notamment parmi les élastomères de silicone émulsionnants, les alkyldiméthicone copolyols et les diméthicone copolyols.

De façon préférentielle, l'émulsionnant siliconé est présent dans la composition de l'invention en une quantité (de matière active) allant par exemple de 0,1 à 10 % en poids, de préférence de 0,5 à 10 % en poids, mieux de 0,5 à 7 % en poids, encore mieux de 1 à 5 % en poids et encore mieux de 1 à 3 % en poids par rapport au poids total de la composition.

L'émulsionnant siliconé est généralement introduit dans la phase huileuse de la composition et fait partie de cette phase huileuse.

### Elastomère de silicone émulsionnant

Par "élastomère de silicone", on entend un organopolysiloxane partiellement ou totalement réticulé, matériau souple et déformable ayant des propriétés viscoélastiques. Son module d'élasticité est tel que ce matériau résiste à la déformation et possède une capacité limitée à l'extension et à la contraction. Ce matériau est capable de retrouver sa forme originelle suite à un étirement.

On entend par « élastomère de silicone émulsionnant » un élastomère de silicone comprenant au moins une chaîne hydrophile, cette chaîne pouvant être notamment oxyalkylénée ou glycérolée. Un élastomère de silicone est un organopolysiloxane élastomère réticulé. L'élastomère de silicone émulsionnant peut donc être choisi parmi les élastomères de silicone comportant au moins une chaîne oxyalkylénée et/ou une chaîne glycérolée.

L'élastomère de silicone comportant au moins une chaîne oxyalkylénée peut être obtenu en particulier par réaction d'addition et réticulation d'un diorganopolysiloxane contenant au moins deux hydrogènes liés chacun à un silicium (A1), et d'un polyoxyalkylène ayant au moins deux groupements à insaturation éthylénique (B1), notamment en présence d'un catalyseur (C1), notamment de catalyseur platine, comme par exemple décrit dans les documents US-A-5,236,986 et US-A-5,412,004.

Le composé (A1) est le composé de base pour la formation d'organopolysiloxane élastomère, et la réticulation s'effectue par réaction d'addition du composé (A1) avec le composé (B1) en présence du catalyseur (C1).

Le composé (B1) est avantageusement un composé oxyéthyléné et/ou oxypropyléné comportant au moins deux groupes vinyliques en position α-ω de la chaîne siliconée, qui vont réagir avec des liaisons Si-H du composé (A1). Le composé (B1) peut être notamment un polyoxyalkylène (notamment polyoxyéthyléne et/ou polyoxypropylène) à terminaisons diméthylvinylsiloxy.

Les groupes organiques liés aux atomes de silicium du composé (A1) peuvent être des groupes alkyles ayant de 1 à 18 atomes de carbone, tels que méthyl, éthyl, propyl, butyl, octyl, décyl, dodécyl (ou lauryl), myristyl, cétyl, stéaryl ; des groupes alkyles substitués tels que 2-phényléthyl, 2-phénylpropyl, 3,3,3-trifluoropropyl ; des groupes aryles tels que phényl, tolyl, xylyl ; des groupes aryles substitués tels que phényléthyl ; et des groupes hydrocarbonés monovalents substitués tels qu'un groupe époxy, un groupe ester carboxylate, ou un groupe mercapto.

Le composé (A1) peut ainsi être choisi parmi les méthylhydrogénopolysiloxanes à terminaisons triméthylsiloxy, les copolymères diméthylsiloxaneméthylhydrogénosiloxane à terminaisons triméthylsiloxy, les copolymères cycliques diméthylsiloxane-méthylhydrogénosiloxane, les copolymères diméthylsiloxane-méthylhydrogénosiloxane-laurylméthylsiloxane à terminaisons triméthylsiloxy.

Le composé (C1) est le catalyseur de la réaction de réticulation, et est notamment l'acide chloroplatinique, les complexes acide chloroplatinique-oléfine, les complexes acide chloroplatinique-alkenylsiloxane, les complexes acide chloroplatinique-dicétone, le platine noir, et le platine sur support.

Le catalyseur (C1) est de préférence ajouté en une quantité de 0,1 à 1000 parts en poids, mieux de 1 à 100 parts en poids, en tant que métal platine propre pour 1000 parts en poids de la quantité totale des composés (A1) et (B1).

En particulier, l'élastomère de silicone comportant au moins une chaîne oxyalkylénée peut être obtenu par réaction de polyoxyalkylène (notamment polyoxyéthyléne et/ou polyoxypropylène) à terminaisons diméthylvinylsiloxy et de méthylhydrogénopolysiloxane à terminaisons triméthylsiloxy, en présence de catalyseur platine.

L'élastomère de silicone comportant au moins une chaîne oxyalkylénée, utilisé selon l'invention, est de préférence un élastomère de silicone comportant au moins une chaîne oxyéthylénée.

En outre, l'élastomère de silicone comportant au moins une chaîne oxyalkylénée est de préférence véhiculé sous forme de gel dans au moins une huile hydrocarbonée et/ou une huile siliconée. Dans ces gels, l'élastomère comportant au moins une chaîne oxyalkylénée est souvent sous forme de particules non-sphériques.

Des élastomères de silicone polyoxyalkylénés sont notamment décrits dans les documents US-A-5,236,986, US-A-5,412,004, US-A-5,837,793, US-A-5,811,487 dont le contenu est incorporé par référence.

Comme élastomères de silicone comportant au moins une chaîne polyoxyéthylénée, on peut utiliser ceux commercialisés par la société Shin Etsu,sous les dénominations :
- KSG-21 (à 27 % en matière active. Nom INCI: Dimethicone /PEG-10 Dimethicone vinyl dimethicone crosspolymer),
- KSG-20 (à 95% en matière active. Nom INCI: PEG-10 Dimethicone Crosspolymer),
- KSG-30 (à 100 % en matière active. Nom INCI : Lauryl PEG-15 Dimethicone vinyl dimethicone crosspolymer),
- KSG-31 (à 25 % en matière active. Nom INCI : Lauryl PEG-15 Dimethicone vinyl dimethicone crosspolymer),
- KSG-32 ou KSG-42 ou KSG-320 ou KSG-30 (à 25 % en matière active. Nom INCI : Lauryl PEG-15 Dimethicone vinyl dimethicone crosspolymer),
- KSG-33 (à 20 % en matière active),
- KSG-210 (à 27 % en matière active. Nom INCI : Dimethicone /PEG-10/15 crosspolymer),
- KSG-31 0 : Polydimethylsiloxane polyoxyéthyléné réticulé modifié lauryle dans de l'huile minérale (mineral oil)
- KSG-330,
- KSG-340,
- X-226146 (à 32 % en matière active. Nom INCI : Dimethicone /PEG-10 Dimethicone vinyl dimethicone crosspolymer),
   ou ceux commercialisés par la société Dow Corning sous les dénominations :
- DC9010 (à 9% en matière active. Nom INCI: PEG-12 dimethicone crosspolymer)
- DC9011 (à 11 % en matière active).

Ces produits se présentent généralement sous forme de gels huileux contenant les particules d'élastomère de silicone.

On utilise de préférence le KSG-210 (Nom INCI : Dimethicone /PEG-10/15 crosspolymer) qui est à environ 27% en matière active d'élastomère de silicone dans de l'huile de silicone.

L'élastomère de silicone émulsionnant peut être également choisi parmi les élastomères de silicone comportant au moins une chaîne glycérolée.

L'élastomère de silicone comportant au moins une chaîne glycérolée peut être obtenu notamment par réaction d'addition et réticulation d'un diorganopolysiloxane contenant au moins un hydrogène lié au silicium (A2) et d'un composé polyglycérolé ayant des groupements à insaturation éthylénique (B2), notamment en présence de catalyseur (C2), en particulier de catalyseur platine.

En particulier, l'organopolysiloxane peut être obtenu par réaction de composé polyglycérolé à terminaisons diméthylvinylsiloxy et de méthylhydrogénopolysiloxane à terminaisons triméthylsiloxy, en présence de catalyseur platine.

Le composé (A2) est le composé de base pour la formation d'organopolysiloxane élastomère, et la réticulation s'effectue par réaction d'addition du composé (A) avec le composé (B2) en présence du catalyseur (C2).

Le composé (A2) est en particulier un organopolysiloxane ayant au moins 2 atomes d'hydrogène liés à des atomes de silicium distincts dans chaque molécule. Le composé (A2) peut avoir une viscosité à 25 °C allant de 1 à 50 000 centistokes, notamment pour être bien miscible avec le composé (B2).

Les groupes organiques liés aux atomes de silicium du composé (A2) peuvent être des groupes alkyles ayant de 1 à 18 atomes de carbone, tels que méthyl, éthyl, propyl, butyl, octyl, décyl, dodécyl (ou lauryl), myristyl, cétyl, stéaryl, ; des groupes alkyles substitués tels que 2-phényléthyl, 2-phénylpropyl, 3,3,3-trifluoropropyl ; des groupes aryles tels que phényl, tolyl, xylyl ; des groupes aryles substitués tels que phényléthyl ; et des groupes hydrocarbonés monovalents substitués tels qu'un groupe époxy, un groupe ester carboxylate, ou un groupe mercapto. De préférence, ledit groupe organique est choisi parmi les groupes méthyl , phényl, lauryl.

Le composé (A2) peut ainsi être choisi parmi les méthylhydrogénopolysiloxanes à terminaisons triméthylsiloxy, les copolymères diméthylsiloxaneméthylhydrogénosiloxane à terminaisons triméthylsiloxy, les copolymères cycliques diméthylsiloxane-méthylhydrogénosiloxane, les copolymères diméthylsiloxane-méthylhydrogénosiloxane-laurylméthylsiloxane à terminaisons triméthylsiloxy.

Le composé (B2) peut être un composé polyglycérolé répondant à la formule (B'2) suivante :

CₘH₂ₘ₋₁-O-[Gly]ₙ-CₘH₂ₘ₋₁ (B'2)

dans laquelle m est un entier allant de 2 à 6, n est un entier allant de 2 à 200, de préférence de 2 à 100, préférentiellement de 2 à 50, mieux de 2 à 20, encore mieux de 2 à 10, et encore mieux de 2 à 5, et en particulier n est égal à 3 ; Gly désigne :

-CH₂-CH(OH)-CH₂-O- ou -CH₂-CH(CH₂OH)-O-

Avantageusement, la somme du nombre de groupements éthyléniques par molécule du composé (B2) et du nombre d'atomes d'hydrogène liés à des atomes de silicium par molécule du composé (A2) est d'au moins 4.

Il est avantageux que le composé (A2) soit ajouté en une quantité telle que le rapport moléculaire entre la quantité totale d'atomes d'hydrogène liés à des atomes de silicium dans le composé (A2) et la quantité totale de tous les groupements à insaturation éthylénique dans le composé (B2) soit compris dans la gamme de 1/1 à 20/1.

Le composé (C2) est le catalyseur de la réaction de réticulation, et est notamment l'acide chloroplatinique, les complexes acide chloroplatinique-oléfine, les complexes acide chloroplatinique-alkenylsiloxane, les complexes acide chloroplatinique-dicétone, le platine noir, et le platine sur support.

Le catalyseur (C2) est de préférence ajouté en une quantité de 0,1 à 1000 parts en poids, mieux de 1 à 100 parts en poids, en tant que métal platine propre pour 1000 parts en poids de la quantité totale des composés (A2) et (B2).

L'élastomère de silicone comportant au moins une chaîne glycérolée, utilisé selon l'invention est généralement mélangé avec au moins une huile hydrocarbonée et/ou une huile siliconée pour former un gel. Dans ces gels, l'élastomère comportant au moins une chaîne glycérolée est souvent sous forme de particules non-sphériques.

De tels élastomères sont notamment décrits dans le document WO-A-2004/024798.

Comme élastomères de silicone comportant au moins une chaîne glycérolée, on peut utiliser ceux vendus par la société Shin Etsu sous les dénominations :
- KSG-710, (à 20-30 % en matière active dans l'huile minérale. Nom INCI : Dimethicone / Polyglycerin-3 Crosspolymer),
- KSG-81 0,
- KSG-820,
- KSG-830,
- KSG-840.

### Alkyldiméthiconecopolyols et diméthicone copolyols

L'émulsionnant siliconé peut être choisi aussi parmi les alkyldiméthicone copolyols et les dimethicone copolyols.

Comme alkyldimethicone copolyols, on peut utiliser par exemple ceux comportant un groupe alkyle en C10-C22, tels que le lauryldiméthicone copolyol comme celui vendu sous la dénomination Q2-5200 par la Société Dow Corning, le cétyldiméthicone copolyol comme celui vendu sous la dénomination ABIL EM 90 par la Société Goldschmidt ou comme le mélange polyglycéryl-4 isostéarate/cétyldiméthicone copolyol/ hexyl laurate vendu sous le nom d'Abil WE 09 par la société Goldschmidt, l'oleyldiméthicone copoylol comme celui vendu sous la dénomination KF-6026 par la Société Shin-Etsu, le stéaryldiméthicone copolyol comme celui vendu sous la dénomination X-22-904 par la Société Shin-Etsu. Il s'agit de préférence du cétyldiméthicone copolyol.

Comme dimethicone copolyols, on peut utiliser par exemple les diméthicone copolyols comportant des groupes oxyéthylénés et des groupes oxypropylénés tel que celui comportant 18 groupes oxyéthylénés et 18 groupes oxypropylénés comme le mélange de cyclomethicone et de dimethicone copolyol, vendu sous les dénominations Dow Corning 3225 C et Dow Corning 5225 C (nom INCI : Cyclopentasiloxane / PEG/PPG-18/18 Dimethicone) par la société Dow Corning, et celui comportant 14 groupes oxyéthylénés et 14 groupes oxypropylénés comme le mélange de dimethicone copolyol et de cyclopentasiloxane (85/15) commercialisé sous la dénomination Abil EM-97 par la société Goldschmidt (nom INCI : Bis-PEG/PPG-14/14 Dimethicone / Cyclopentasiloxane). On peut aussi utiliser les diméthicone copolyols ne comportant que des groupes oxyéthylénés, tels que les produits commercialisés sous les dénominations commerciales KF-6015 ou KF-6017 (PEG-10 dimethicone) par la société Shin-Etsu.

Quand l'émulsionnant siliconé est un alkyldiméthicone copolyol ou un dilmethicone copoyol, la composition peut contenir une petite quantité d'un émulsionnant non siliconé tel qu'un ester d'acide gras et de glycéryle comme le Polyglycéryl isostearate qui est par exemple dans le mélange Abil WE 09 cité ci-dessus. Dans ce cas, la quantité d'ester d'acide gras et de glycéryle est en une quantité allant de 0,2 à 5 % en poids et mieux de 0,5 à 2 % en poids par rapport au poids total de la composition.

### Polymère hydrophile

La composition contient un ou plusieurs polymères hydrophiles choisis parmi les polymères acryliques réticulés et les polymères d'acide 2-acrylamido 2-méthylpropane sulfonique.

La quantité de polymère(s) hydrophile(s) dans la composition de l'invention peut aller par exemple, en matière active, de 0,1 à 5 % en poids, de préférence de 0,2 à 5 % en poids, mieux de 0,2 à 3 % en poids par rapport au poids total de la composition.

### Polymères acryliques réticulés

Comme polymères acryliques réticulés utilisables dans la composition selon l'invention, on peut citer notamment les polymères carboxyvinyliques modifiés ou non, tels que les copolymères d'acide acrylique et d'acrylate ou methacrylate d'alkyle en C10-C30 comme les produits commercialisés sous les dénominations Carbopol (nom INCI : carbomer) et Pemulen (nom INCI : Acrylates/C10-30 akyl acrylate crosspolymer) par la société Goodrich, ou tel que le polyacrylate de sodium réticulé commercialisé sous la dénomination Cosmedia SP par la société Cognis (nom INCI : Sodium polyacrylate) ou le Norsocryl S35 vendu par la société ATOFINA.

Parmi les polymères acryliques réticulés, on utilise de préférence le polyacrylate de sodium (Cosmedia SP).

### Polymères d'acide 2-acrylamido 2-méthylpropane sulfonique

On entend dans la présente demande « polymère comportant des motifs acide 2-acrylamido 2-méthylpropane sulfonique » (AMPS), aussi bien des homopolymères que des copolymères, et aussi bien des polymères réticulés que des polymères non réticulés.

Ce sont des polymères hydrosolubles ou hydrodispersibles ou gonflables dans l'eau.

De façon préférentielle, les polymères d'AMPS utilisés conformément à l'invention sont neutralisés partiellement ou totalement par une base minérale (soude, potasse, ammoniaque) ou une base organique telle que la mono-, di- ou tri-éthanolamine, un aminométhylpropanediol, la N-méthyl-glucamine, les acides aminés basiques comme l'arginine et la lysine, et les mélanges de ces composés. Ils sont généralement neutralisés. On entend dans la présente invention par « neutralisés », des polymères totalement ou pratiquement totalement neutralisés, c'est-à-dire neutralisés à au moins 90 %.

Les polymères d'AMPS utilisés dans la composition de l'invention ont généralement un poids moléculaire moyen en nombre allant de 1000 à 20 000 000 g/mole, de préférence allant de 20 000 à 5 000 000 et plus préférentiellement encore de 100 000 à 1 500 000 g/mole.

Ces polymères selon l'invention peuvent être réticulés ou non réticulés.

Lorsque les polymères sont réticulés, les agents de réticulation peuvent être choisis parmi les composés à polyinsaturation oléfinique couramment utilisés pour la réticulation des polymères obtenus par polymérisation radicalaire.

On peut citer par exemple comme agents de réticulation, le divinylbenzène, l'éther diallylique, le dipropylèneglycol-diallyléther, les polyglycol-diallyléthers, le triéthylèneglycol-divinyléther, l'hydroquinone-diallyl-éther, le di(méth)acrylate de d'éthylèneglycol ou de tétraéthylèneglycol, le triméthylol propane triacrylate, le méthylène-bis-acrylamide, le méthylène-bis-méthacrylamide, la triallylamine, le triallylcyanurate, le diallylmaléate, la tétraallyléthylènediamine, le tétra-allyloxyéthane, le triméthylolpropane-diallyléther, le (méth)acrylate d'allyle, les éthers allyliques d'alcools de la série des sucres, ou d'autres allyl- ou vinyl- éthers d'alcools polyfonctionnels, ainsi que les esters allyliques des dérivés de l'acide phosphorique et/ou vinylphosphonique, ou les mélanges de ces composés.

Selon un mode préféré de réalisation de l'invention, l'agent de réticulation est choisi parmi le méthylène-bis-acrylamide, le méthacrylate d'allyle ou le triméthylol propane triacrylate (TMPTA). Le taux de réticulation va en général de 0,01 à 10 % en mole et plus particulièrement de 0,2 à 2 % en mole par rapport au polymère.

Les homopolymères d'AMPS préférés pour être utilisés dans la composition de l'invention sont réticulés et neutralisés, et ils peuvent être obtenus selon le procédé de préparation comprenant les étapes suivantes :
(a) on disperse ou on dissout l'acide 2-acrylamido 2-méthylpropane sulfonique sous forme libre dans une solution de tertio-butanol ou d'eau et de tertio-butanol ;
(b) on neutralise la solution ou la dispersion de monomère obtenue en (a) par une ou plusieurs bases minérales ou organiques, de préférence l'ammoniaque NH₃, dans une quantité permettant d'obtenir un taux de neutralisation des fonctions acides sulfoniques du polymère allant de 90 à 100% ;
(c) on ajoute à la solution ou dispersion obtenue en (b), le ou les monomères réticulants ;
(d) on effectue une polymérisation radicalaire classique en présence d'amorceurs de radicaux libres à une température allant de 10 à 150°C ; le polymère précipitant dans la solution ou la dispersion à base de tertio-butanol.

Les homopolymères d'AMPS plus particulièrement préférés comprennent, distribués de façon aléatoire :
a) de 90 à 99,9% en poids de motifs de formule générale (I) suivante : 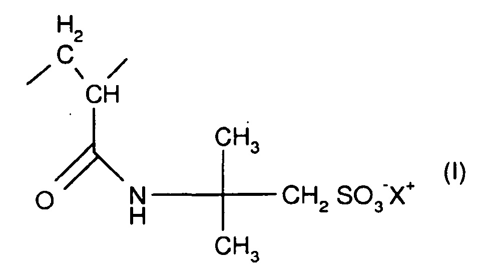 dans laquelle X⁺ désigne un proton, un cation de métal alcalin, un cation alcalinoterreux ou l'ion ammonium, au plus 10% mol des cations X⁺ pouvant être des protons H⁺ ;
b) de 0,01 à 10% en poids de motifs réticulants provenant d'au moins un monomère ayant au moins deux double-liaison oléfiniques ; les proportions en poids étant définis par rapport au poids total du polymère.

Les homopolymères utilisés selon l'invention et plus particulièrement préférés comprennent de 98 à 99,5 % en poids de motifs de formule (I) et de 0,2 à 2 % en poids de motifs réticulants.

Comme homopolymère de ce type, on peut citer notamment l'homopolymère réticulé et neutralisé d'acide 2-acrylamido 2-méthylpropane sulfonique, commercialisé par la société Clariant sous la dénomination commerciale « Hostacerin AMPS » (nom CTFA : ammonium polyacryldimethyltauramide).

Les copolymères d'AMPS utilisables dans la composition de l'invention peuvent être choisis notamment parmi :
1) les copolymères anioniques réticulés d'acrylamide ou méthylacrylamide et d'acide 2-acrylamido-2-méthylpropane sulfonique, notamment ceux se présentant sous la forme d'une émulsion E/H, tels que ceux commercialisés sous le nom de SEPIGEL 305 par la société Seppic (nom C.T.F.A. : Polyacrylamide / C13-14 Isoparaffin / Laureth-7), sous le nom de SIMULGEL 600 par la société Seppic (nom C.T.F.A. : Acrylamide / Sodium acryloyldimethyltaurate copolymer / Isohexadecane / Polysorbate 80),
2) les copolymères d'acide (méth)acrylique ou de (méth)acrylate et d'acide 2-acrylamido- 2-méthylpropane sulfonique, notamment ceux se présentant sous la forme d'une émulsion E/H, tels que ceux commercialisés sous le nom de SIMULGEL NS par la société Seppic (copolymère d'acrylamido-2-methyl propane sulfonate de sodium / hydroxyethyl acrylate en émulsion inverse à 40% dans Polysorbate 60 et squalane) (nom CTFA : hydroxyethyl acrylate/sodium acryloyldimethyltaurate copolymer / squalane / polysorbate 60) ou ceux commercialisés sous le nom de SIMULGEL EG par la société Seppic (copolymère d'acide acrylique / acrylamido-2-methylpropane-sulfonique sous forme de sel de sodium en émulsion inverse à 45% dans isohexadecane / eau) (nom C.T.F.A. : Sodium Acrylate / Sodium acryloydimethyl-taurate copolymer / Isohexadecane / Polysorbate 80), ;
3) les copolymères d'acide 2-acrylamido-2-méthylpropane sulfonique et de vinylpyrrolidone ou de vinylformamide, tels que les produits commercialisés sous les dénominations ARISTOFLEX AVC par la société Clariant,
4) les copolymères d'AMPS à motif hydrophobe, notamment les copolymères comportant :
   - 80 à 99 % en moles et de préférence de 85 à 99 % en moles de motifs acide 2-acrylamido 2-méthylpropane sulfonique (AMPS) de formule (I) telle que définie ci-dessus et
   - 1 à 20 % en moles et de préférence de 1 à 15 % en moles de motifs hydrophobes de formule (II) suivante : 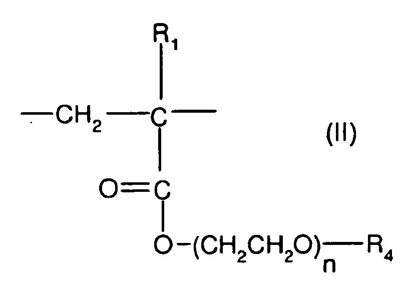
dans laquelle n désigne un nombre entier variant de 3 à 100, de préférence de 3 à 50 et plus préférentiellement de 7 à 25 ; R₁ est l'hydrogène ou un radical méthyle, et R₄ désigne un radical alkyle linéaire ou ramifié comportant de 6 à 22 atomes de carbone, de préférence de 10 à 22 atomes de carbone et mieux de 14 à 22 atomes de carbone.

Comme copolymères d'AMPS à motif hydrophobe, on peut citer notamment le copolymère d'AMPS et de méthacrylate d'alcool en C₁₂-C₁₄ éthoxylé (copolymère non réticulé obtenu à partir de Genapol LA-070 et d'AMPS) (nom CTFA : Ammonium Acryloyldimethyltaurate / Laureth-7 Methacrylate Copolymer) commercialisé sous la dénomination ARISTOFLEX LNC par la société Clariant, et le copolymère d'AMPS et de méthacrylate de stéaryle éthoxylé (25 EO) (copolymère réticulé par le triméthylolpropane triacrylate, obtenu à partir de Genapol T-250 et d'AMPS) (nom CTFA : Ammonium Acryloyldimethyltaurate / Steareth-25 Methacrylate Crosspolymer) commercialisé sous la dénomination ARISTOFLEX HMS par la société Clariant.

### Charge à base de silicium

La composition peut contenir une ou plusieurs charges à base de silicium. Ces charges peuvent être choisies parmi les silices ayant un diamètre allant de 10 nm à 29 microns et les particules concaves ou annulaires de matériau siliconé.

La ou les charges à base de silicium peuvent être présentes dans la composition selon l'invention en une teneur allant de 0,1 à 10 % en poids, de préférence allant de 0,5 à 7 % en poids, et préférentiellement allant de 0,5 à 5 % en poids par rapport au poids total de la composition.

### Silices

Le diamètre des silices utilisés va de 10 nm à 20 microns, et les silices sont de préférence des silices poreuses ayant une surface spécifique supérieure à 70m²/g.

Comme silices, on peut utiliser en particulier :
- des silices pyrogénées hydrophiles telles que celle commercialisée sous la dénomination AEROSIL 200 par la société Degussa ;
- des microsphères de silice poreuse, telles que celles commercialisées sous les dénominations SB 700 par la société Miyoshi ;
- des silices précipitées non traitées ou traitées en surface, par exemple traitée par une cire minérale, comme celle commercialisée sous la dénomination « Acematt OK 412 » par la société Degussa.

### Particules concaves ou annulaires de matériau siliconé

Les particules concaves ou annulaires présentes dans la composition selon l'invention sont des particules siliconées, en particulier des particules de portions de sphères creuses constituées d'un matériau siliconé.

Lesdites particules ont de préférence un diamètre moyen inférieur ou égal à 10 µm, notamment allant de 0,1 µm à 8 µm, préférentiellement de 0,2 à 7 µm, plus préférentiellement allant de 0,5 à 6 µm, et de préférence encore allant de 0,5 à 4 µm.

Par diamètre moyen, on entend la plus grande dimension de la particule.

De façon avantageuse, ces particules ont une densité supérieure à 1.

Les portions de sphères creuses utilisées dans la composition selon l'invention peuvent avoir la forme de sphères creuses tronquées, présentant un seul orifice communiquant avec leur cavité centrale, et ayant une section transversale en forme de fer à cheval ou d'arceau.

Le matériau siliconé est un polysiloxane réticulé de structure tridimensionnelle ; il comprend de préférence, voire est constitué de, des motifs de formule (I) SiO₂ et de formule (II) R¹SiO_{1,5}
dans lesquels R¹ désigne un groupe organique ayant un atome de carbone directement relié à l'atome de silicium.

Le groupe organique R¹ peut être un groupe organique réactif ; R¹ peut être plus particulièrement un groupe époxy, un groupe (méth)acryloxy, un groupe alkényle, un groupe mercaptoalkyle, aminoalkyle, halogénoalkyle, un groupe glycéroxy, un groupe uréïdo, un groupe cyano, et de préférence, un groupe époxy, un groupe (méth)acryloxy, un groupe alkényle, un groupe mercaptoalkyle, aminoalkyle. Ces groupes comprennent généralement de 2 à 6 atomes de carbone, notamment de 2 à 4 atomes de carbone.

Le groupe organique R¹ peut être aussi un groupe organique non réactif ; R¹ peut être alors plus particulièrement un groupe alkyle en C₁-C₄, notamment un groupe méthyle, éthyle, propyle, butyle, ou un groupe phényle, et de préférence un groupe méthyle.

Comme groupe époxy, on peut citer un groupe 2-glycidoxyéthyle, un groupe 3-glycidoxypropyle, un groupe 2-(3,4-époxycyclohexyl)propyle.

Comme groupe (méth)acryloxy, on peut citer un groupe 3-méthacryloxypropyle, un groupe 3-acryloxypropyle.

Comme groupe alkényle, on peut citer les groupes vinyle, allyle, isopropényle.

Comme groupe mercaptoalkyle, on peut citer les groupes mercaptopropyle, mercaptoéthyle.

Comme groupe aminoalkyle, on peut citer un groupe 3-(2-aminoéthyl)aminopropyle, un groupe 3-aminopropyle, un groupe N,N-diméthylaminopropyle.

Comme groupe halogénoalkyle, on peut citer un groupe 3-chloropropyle, un groupe trifluoropropyle.

Comme groupe glycéroxy, on peut citer un groupe 3-glycéroxypropyle, un groupe 2-glycéroxyéthyle.

Comme groupe uréido, on peut citer un groupe 2-uréidoéthyle.

Comme groupe cyano, on peut citer les groupes cyanopropyle, cyanoéthyle.

De préférence, dans le motif de formule (II), R¹ désigne un groupe méthyle.

Avantageusement, le matériau siliconé comprend les motifs (I) et (II) selon un rapport molaire motif (I) / motif (II) allant de 30/70 à 50/50, de préférence allant de 35/65 à 45/55.

Les particules de matériau siliconé peuvent être notamment susceptibles d'être obtenues selon un procédé comprenant :
(a) l'introduction dans un milieu aqueux, en présence d'au moins un catalyseur d'hydrolyse, et éventuellement d'au moins un tensioactif, d'un composé (III) de formule SiX₄ et d'un composé (IV) de formule RSiY₃, où X et Y désignent indépendamment l'un de l'autre un groupe alcoxy en C₁-C₄, un groupe alcoxyéthoxy renfermant un groupement alcoxy en C₁-C₄, un groupe acyloxy en C₂-C₄, un groupe N,N-dialkylamino renfermant des groupements alkyle en C₁-C₄, un groupe hydroxyle, un atome d'halogène ou un atome d'hydrogène, et R désigne un groupe organique comportant un atome de carbone directement relié à l'atome de silicium ; et
(b) la mise en contact du mélange résultant de l'étape (a) avec une solution aqueuse renfermant au moins un catalyseur de polymérisation et éventuellement au moins un tensioactif, à une température comprise entre 30 et 85°C, pendant au moins deux heures.

L'étape (a) correspond à une réaction d'hydrolyse et l'étape (b) correspond à une réaction de condensation.

Dans l'étape (a), le rapport molaire du composé (III) au composé (IV) va habituellement de 30/70 à 50/50, avantageusement de 35/65 à 45/45, et est préférentiellement de 40/60. Le rapport en poids de l'eau au total des composés (III) et (IV) va de préférence de 10/90 à 70/30. L'ordre d'introduction des composés (III) et (IV) dépend généralement de leur vitesse d'hydrolyse. La température de la réaction d'hydrolyse va généralement de 0 à 40 °C et ne dépasse habituellement pas 30°C pour éviter une condensation prématurée des composés.

### Pour les groupements X et Y des composés (III) et (IV) :

Comme groupe alcoxy en C₁-C₄, on peut citer les groupes méthoxy, éthoxy ;

Comme groupe alkoxyéthoxy renfermant un groupe alcoxy en C₁-C₄, on peut citer les groupes méthoxyéthoxy, butoxyéthoxy ;

Comme groupe alcoxy en C₂-C₄, on peut citer les groupes acétoxy, propioxy;

Comme groupe N,N-dilakylamino renfermant des groupements alkyle en C₁-C₄, on peut citer les groupes diméthylamino, diéthylamino ;

Comme atome d'halogène, on peut citer les atomes de chlore, de brome.

Comme composés de formule (III), on peut citer le tétraméthoxysilane, le tétraéthoxysilane, le tétrabutoxysilane, le triméthoxyéthoxysilane, le tributoxyéthoxysilane, le tétraacétoxysilane, le tétrapropioxysilane, le tétraacétoxysilane, le tétra(diméthylamino)silane, le tétra(diéthylamino)silane, le silane tétraol, le chlorosilane triol, le dichlorodisilanol, le tétrachlorosilane, le chlorotrihydrogénosilane. De préférence, le composé de formule (III) est choisi parmi le tétraméthoxysilane, le tétraéthoxysilane, le tétrabutoxysilane, et leurs mélanges.

Le composé de formule (III) conduit après la réaction de polymérisation à la formation des motifs de formule (1).

Le composé de formule (IV) conduit après la réaction de polymérisation à la formation des motifs de formule (II).

Le groupe R dans le composé de formule (IV) a la signification telle que décrite pour le groupe R¹ pour le composé de formule (II).

Comme exemples de composés de formule (IV) comportant un groupe R organique non réactif, on peut citer le méthyltriméthoxysilane, l'éthyltriéthoxysilane, le propyltributoxysilane, le butyltributoxysilane, le phényltriméthoxyéthoxysilane, le méthyl tributoxyethoxysilane, le méthyltriacétoxysilane, le méthyltripropioxysilane, le méthyltriacétoxysilane, le méthyltri(diméthylamino)silane, le méthyltri(diéthylamino)silane, le méthylsilane triol, le méthylchlorodisilanol, le méthyltrichlorosilane, le méthyltrihydrogénosilane.

Comme exemples de composés de formule (IV) comportant un groupe R organique réactif, on peut citer :
- les silanes ayant un groupe époxy comme le 3-glycidoxypropyl triméthoxysilane, le 3-glycidoxypropyl triéthoxysilane, le 2-(3,4-époxycyclohexyl)éthyl triméthoxysilane, le 3-glycidoxypropylméthyl diméthoxysilane, le 3-glycidoxypropylméthyl diméthoxysilane, le 2-glycidoxyéthylméthyldiméthoxysilane, le 3-glycidoxypropyl diméthylméthoxysilane, le 2-glycidoxyéthyl diméthylméthoxysilane ;
- les silanes ayant un groupe (méth)acryloxy comme le 3-méthacryloxypropyl triméthoxysilane, le 3-acryloxypropyl triméthoxysilane ;
- les silanes ayant un groupe alkényle comme le vinyl triméthoxysilane, l'allyl triméthoxysilane, l'isopropényl triméthoxysilane ;
- les silanes ayant un groupe mercapto comme le mercaptopropyl triméthoxysilane, le mercaptoéthyl triméthoxysilane ;
- les silanes ayant un groupe aminoalkyle comme le 3-aminopropyl triméthoxysilane, le 3-(2-aminoéthyl)aminopropyl triméthoxysilane, le N,N-diméthylaminopropyl triméthoxysilane, le N,N-diméthylaminoéthyl triméthoxysilane ;
- les silanes ayant un groupe halogénoalkyl comme le 3-chloropropyl triméthoxysilane, le trifluoropropyl triméthoxysilane ;
- les silanes ayant un groupe glycéroxy comme le 3-glycéroxypropyl triméthoxysilane, le di(3-glycéroxypropyl)diméthoxysilane ;
- les silanes ayant un groupe uréido comme le 3-uréïdopropyl triméthoxysilane, le 3-uréïdopropyl méthyldiméthoxysilane, le 3-uréïdopropyl diméthyl-méthoxysilane ;
- les silanes ayant un groupe cyano comme le cyanopropyl triméthoxysilane, le cyanopropyl méthyldiméthoxysilane, le cyanopropyl diméthylméthoxysilane.

De préférence, le composé de formule (IV) comportant un groupe R organique réactif est choisi parmi les silanes ayant un groupe époxy, les silanes ayant un groupe (méth)acryloxy, les silanes ayant un groupe alkényle, les silanes ayant un groupe mercapto, les silanes ayant un groupe aminoalkyle.

Des exemples de composés (III) et (IV) préférés pour la mise en oeuvre de cette invention sont respectivement le tétraéthoxysilane et le méthyltriméthoxysilane.

Comme catalyseurs d'hydrolyse et de polymérisation, on peut utiliser indépendamment des catalyseurs basiques tels que l'hydroxyde de sodium, l'hydroxyde de potassium, le carbonate de sodium, l'hydrogénocarbonate de sodium, l'ammoniaque ou des amines telles que la triméthylamine, la triéthylamine, l'hydroxyde de tétraméthylammonium, ou des catalyseurs acides tels que les acides organiques comme l'acide citrique, l'acide acétique, l'acide méthanesulfonique, l'acide p-toluène sulfonique, l'acide dodécylbenzènesulfonique, l'acide dodécylsulfonique, ou les acides minéraux tels que l'acide chlorhydrique, l'acide sulfurique, l'acide phosphorique.

Lorsqu'il est présent, le tensioactif utilisé est de préférence un tensioactif non ionique ou anionique ou un mélange des deux. Le dodécylbenzènesulfonate de sodium peut être utilisé comme tensioactif anionique. La fin de l'hydrolyse est marquée par la disparition des produits (III) et (IV), insolubles dans l'eau, et l'obtention d'une couche liquide homogène.

L'étape (b) de condensation peut utiliser le même catalyseur que l'étape d'hydrolyse ou un autre catalyseur choisi parmi ceux mentionnés précédemment.

A l'issue de ce procédé, on obtient une suspension dans l'eau de fines particules organosiliconées qui peuvent éventuellement être ensuite séparées de leur milieu. Le procédé décrit ci-dessus peut donc comprendre une étape supplémentaire de filtration, par exemple sur filtre à membrane, du produit résultant de l'étape (b), suivie éventuellement d'une étape de centrifugation du filtrat destinée à séparer les particules du milieu liquide, puis d'une étape de séchage des particules. D'autres méthodes de séparation peuvent bien entendu être employées.

La forme des portions de sphères creuses obtenues selon le procédé ci-dessus, ainsi que leurs dimensions, dépendront notamment du mode de mise en contact des produits à l'étape (b).

Un pH plutôt basique et une introduction à froid du catalyseur de polymérisation dans le mélange issu de l'étape (a) conduira à des portions de sphères creuses en forme de "bols" à fond arrondi, tandis qu'un pH plutôt acide, et une introduction goutte-à-goutte du mélange issu de l'étape (a) dans le catalyseur de polymérisation chaud, conduira à des portions de sphères creuses ayant une section transversale en forme de "fer à cheval".

Selon un mode de réalisation préféré de l'invention, on utilise des portions de sphères creuses en forme de "bols". Celles-ci peuvent être obtenues comme décrit dans la demande JP-A-2003-128788.

Des portions de sphères creuses en forme de fer à cheval sont décrites dans la demande JP-A-2000-191789.

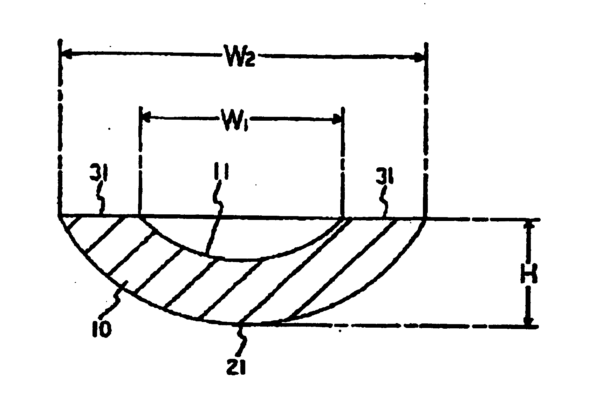

La figure 1 annexée illustre une particule concave sous forme de portions de sphères en forme de bol en coupe transversale. La largeur W2 correspond au diamètre des particules.

Comme il ressort de cette figure, ces portions concaves sont formées (en coupe perpendiculaire à un plan de l'ouverture délimitée par la portion de sphère creuse) d'un petit arc interne (11), d'un grand arc externe (21) et de segments (31) qui relient les extrémités des arcs respectifs, la largeur (W1) entre les deux extrémités du petit arc interne (11) allant de 0,01 à 8 µm, de préférence de 0,02 à 6 µm en moyenne, la largeur (W2) entre les eux extrémités du grand arc externe (21) allant de 0,05 à 10 µm, de préférence de 0,06 à 8 µm en moyenne et la hauteur (H) du grand arc externe (21) allant de 0,015 à 8 µm, de préférence de 0,03 à 6 µm en moyenne.

Les dimensions mentionnées ci-dessus sont obtenues en calculant la moyenne des dimensions de cent particules choisies sur une image obtenue au microscope électronique à balayage.

Comme particules concaves sous forme de portions de sphères utilisables selon l'invention, on peut citer par exemple :
- les particules constituées de l'organosilicone réticulé TAK-110 (polymère réticulé méthylsilanol/silicate) de la société TAKEMOTO OIL & FAT, en forme de bol, de largeur 2,5 µm, de hauteur 1,2 µm et d'épaisseur 150 nm (particules vendues sous la dénomination NLK-506 par la société Takemoto Oil & Fat) ;
- les particules constituées de l'organosilicone réticulé TAK-110 (polymère réticulé méthylsilanol/silicate) de la société TAKEMOTO OIL & FAT, en forme de bol, de largeur 0,8 µm, de hauteur 0,4 µm et d'épaisseur 130 nm (particules vendues sous la dénomination NLK-515 par la société Takemoto Oil & Fat) ;
- les particules constituées de l'organosilicone réticulé TAK-110 (polymère réticulé méthylsilanol/silicate) de la société TAKEMOTO OIL & FAT, en forme de bol, de largeur 7 µm, de hauteur 3,5 µm et d'épaisseur 200 nm (particules vendues sous la dénomination NLK-510 par la société Takemoto Oil & Fat).

Ces particules ont pour nom INCI : Methylesilanol/silicate crosspolymer.

Avantageusement, les particules concaves siliconées ont un diamètre moyen inférieur ou égal à 5 µm, notamment allant de 0,1 µm à 5 µm, préférentiellement allant de 0,2 à 5 µm, plus préférentiellement allant de 0,5 à 4 µm, et de préférence encore allant de 0,5 à 3 µm.

Ces particules permettent, outre la réduction voire l'élimination du toucher collant, l'optimisation des propriétés de glissant, d'étalement, et de confort de la composition selon l'invention.

Les particules siliconées de forme annulaire sont de préférence choisies parmi celles décrites et synthétisées dans la demande US-A-2006/0089478. Elles présentent un diamètre moyen extérieur de 0,05 à 15 µm et un diamètre moyen intérieur de 001 à 10 µm; la différence entre le diamètre moyen extérieur et le diamètre moyen intérieur étant de 0,04 à 5 µm.

Elles présentent un réseau polysiloxane comprenant des unités siloxane de formule (1), (2) (3) (4) (5) et (6) :

SiO_{4/2} (1)

Si(OH)_{3/2} (2)

R¹SiO_{3/2} (3)

R²SiO_{3/2} (4)

R³(SiOH)_{2/2} (5)

R⁴ (SiOH)_{2/2} (6)

dans lesquelles
- R¹ et R³ désignent des groupes hydrocarbonés non réactifs, notamment des groupes alkyle, cycloalkyle, aryle, alkylaryle ou aralkyle, de préférence les groupes alkyles en C₁-C₃, notamment méthyle, éthyle, propyle, et préférentiellement un groupe méthyle ;
- R² et R⁴ désignent chacun un groupe hydrocarboné choisi parmi les groupes acryloxy, methacryloxy, vinyle ou mercapto ;
- le rapport molaire unités siloxane de formule (1) / unités siloxane de formule (2), (3), (4), (5) et (6) étant de 20/80 à 50/50 ;
- le rapport molaire unités siloxane de formule (2), (3) et (4) /unités siloxane de formule (5) et (6) étant de 50/50 à 75/25;
- le rapport molaire unités siloxane de formule (3) et (5) /unités siloxane de formule (4) et (6) étant de 20/80 à 60/40.

Comme groupe acryloxy, on peut citer un groupe 2-méthacryloxyéthyle, un groupe 3-acryloxypropyl.

Comme groupe (méth)acryloxy, on peut citer un groupe 3-méthacryloxypropyl, un groupe 3-acryloxypropyle.

Comme groupe mercaptoalkyle, on peut citer un groupe mercaptopropyl, mercaptoéthyl.

Comme groupe vinyle, on peut citer les groupes allyle, isopropenyle, 2-methylallyle.

Selon un mode préféré de réalisation de l'invention, les charges à base de silicium sont choisies parmi les particules concaves ou annulaires de matériau siliconé, qui apportent un bon effet matifiant et de bonnes propriétés cosmétiques à la composition.

### Phase huileuse

La phase huileuse est constituée des huiles et de tous les autres corps gras et constituants lipophiles pouvant être présents dans la composition de l'invention, y compris l'élastomère de silicone et le polymère lipophile. Toutes les huiles cosmétiquement acceptables peuvent être utilisées.

On entend par "huile" un corps gras liquide à la température ambiante (25°C). Comme huiles utilisables dans la composition de l'invention, on peut citer par exemple :
- les huiles hydrocarbonées d'origine animale, telles que le perhydrosqualène et le squalane ;
- les huiles hydrocarbonées d'origine végétale, telles que les triglycérides liquides d'acides gras comportant de 4 à 10 atomes de carbone comme les triglycérides des acides heptanoïque ou octanoïque ou encore, par exemple les huiles de tournesol, de maïs, de soja, de courge, de pépins de raisin, de sésame, de noisette, d'abricot, de macadamia, d'arara, de tournesol, de ricin, d'avocat, les triglycérides des acides caprylique/caprique comme ceux vendus par la société Stearineries Dubois ou ceux vendus sous les dénominations Miglyol 810, 812 et 818 par la société Dynamit Nobel, l'huile de jojoba, l'huile de beurre de karité;
- les esters et les éthers de synthèse, notamment d'acides gras, comme les huiles de formules R¹COOR² et R¹OR² dans laquelle R¹ représente le reste d'un acide gras comportant de 8 à 29 atomes de carbone, et R² représente une chaîne hydrocarbonée, ramifiée ou non, contenant de 3 à 30 atomes de carbone, comme par exemple l'huile de Purcellin, l'isononanoate d'isononyle, le myristate d'isopropyle, le palmitate d'éthyl-2-hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéaryl-malate, le citrate de triisocétyle, les heptanoates, octanoates, décanoates d'alcools gras ; les esters de polyol, comme le dioctanoate de propylène glycol, le diheptanoate de néopentylglycol et le diisononanoate de diéthylèneglycol ; et les esters du pentaérythritol comme le tétraisostéarate de pentaérythrityle (Prisorine 3631) ;
- les hydrocarbures linéaires ou ramifiés, d'origine minérale ou synthétique, tels que les huiles de paraffine, volatiles ou non, et leurs dérivés, la vaseline, les polydécènes, le polyisobutène hydrogéné tel que l'huile de Parléam ;
- les huiles de silicone, comme par exemple les polyméthylsiloxanes (PDMS) volatiles ou non à chaîne siliconée linéaire ou cyclique, liquides ou pâteux à température ambiante, notamment les cyclopolydiméthylsiloxanes (cyclométhicones) telles que la cyclohexasiloxane et la cyclopentasiloxane ; les polydiméthylsiloxanes (ou diméthicones) comportant des groupements alkyle, alcoxy ou phényle, pendant ou en bout de chaîne siliconée, groupements ayant de 2 à 24 atomes de carbone ; les silicones phénylées comme les phényltriméthicones, les phényldiméthicones, les phényltriméthylsiloxydiphénylsiloxanes, les diphényl-diméthicones, les diphénylméthyldiphényl trisiloxanes, les 2-phényléthyltriméthyl-siloxysilicates, et les polyméthylphénylsiloxanes ;
- les alcools gras ayant de 8 à 26 atomes de carbone, comme l'alcool cétylique, l'alcool stéarylique et leur mélange (alcool cétylstéarylique) ;
- les huiles fluorées partiellement hydrocarbonées et/ou siliconées comme celles décrites dans le document JP-A-2-295912 ;
- et leurs mélanges.

On entend ci-dessus par « huile hydrocarbonée », toute huile comportant majoritairement des atomes de carbone et d'hydrogène, et éventuellement des groupements ester, éther, fluoré, acide carboxylique et/ou alcool.

En plus des huiles indiquées ci-dessus, la composition de l'invention peut contenir d'autres corps gras dans la phase huileuse, tels que les acides gras comportant de 8 à 30 atomes de carbone, comme l'acide stéarique ; les résines de silicone telles que la trifluorométhyl-C1-4-alkyldimethicone et la trifluoropropyldimethicone ; les gommes de silicone (dimethiconol), les élastomères de silicone non émulsionnants, comme les produits commercialisés sous les dénominations « KSG 6» ou « KSG 16» par la société Shin-Etsu, sous les dénominations « Trefil », « BY29 » ou « EPSX » par la société Dow Corning ou sous les dénominations « Gransil » par la société Grant Industries ; les cires, par exemple les cires minérales, les cires d'origine animale comme la cire d'abeille, les cires d'origine végétale, les huiles hydrogénées concrètes à 25°C, les esters gras et les glycérides concrets à 25°C, les cires synthétiques, les cires de silicone ; et leurs mélanges.

La phase huileuse peut être présente dans la composition selon l'invention en une quantité allant de 5 à 40 % et de préférence de 10 à 30 % en poids par rapport au poids total de la composition.

Selon un mode préféré de réalisation de l'invention, la phase huileuse contient un ou plusieurs épaississants huileux. Cet épaississant peut être choisi par exemple parmi les argiles organomodifiées qui sont des argiles traitées par des composés choisis notamment parmi les amines quaternaires et les amines tertiaires. Comme argiles organomodifiées, on peut citer les bentonites organomodifiées telles que celles vendues sous les dénominations Bentone par la société RHEOX, comme celles modifiées par chlorure de distéaryl di-méthyl ammonium (Bentone 38 et Bentone 34), ou celle modifiée par du chlorure de stearyl benzyl dimethyl ammonium (Bentone 27).

L'épaississant de phase huileuse peut être choisi aussi parmi les esters d'acide gras et de glycérine, et en particulier les triesters de glycérine comme le tristéarate de glycéryle (tristearine), tels que le mélange de stéarate de glycol acétylé et de tristéarate de glycéryle, commercialisé sous la dénomination Unitwix par la société United Guardian.

L'épaississant de phase huileuse peut être choisi encore parmi les esters d'acide gras et de dextrine, comme notamment le palmitate de dextrine, notamment ceux commercialisés sous les dénominations Rheopearl par la société Chiba Flour Milling.

A la place de cet épaississant ou en plus de cet épaississant, la phase huileuse peut contenir une ou plusieurs cires choisies parmi celles décrites ci-dessus, et notamment une cire synthétique telle que la cire de polyméthylène ou la cire de polyéthylène, ou bien un ou plusieurs corps gras pâteux tels que la vaseline.

Le ou les épaississants peuvent être présents en une quantité allant par exemple de 0,1 à 5 % en poids, par rapport au poids total de la composition, de préférence de 0,1 à 3 % en poids et mieux de 0,2 à 2% en poids par rapport au poids total de la composition.

### Phase aqueuse

La quantité de phase aqueuse peut aller de 60 à 95 % en poids, de préférence de 70 à 90 % en poids et mieux 70 à 85 % en poids par rapport au poids total de la composition.

La phase aqueuse comprend de l'eau et les adjuvants (actifs ou additifs) hydrophiles. L'eau représente de préférence au moins 30 % du poids total de la composition, de préférence au moins 40 % en poids, et mieux d'au moins 50 % en poids. Cette quantité peut aller par exemple de 30 à 90 %, de préférence de 40 à 80 % en poids et mieux de 50 à 75 % en poids par rapport au poids total de la composition.

Comme adjuvants hydrophiles, on peut citer notamment les monoalcools comportant 2 à 8 atomes de carbone, comme l'éthanol et l'isopropanol, et les polyols comme la glycérine, les glycols comme le pentylène glycol, le propylène glycol, le butylène glycol, l'isoprène glycol et les polyéthylène glycols tels que le PEG-8 ; le sorbitol ; les sucres tels que le glucose, le fructose, le maltose, le lactose, le sucrose ; et leurs mélanges.

### Adjuvants

De façon connue, la composition de l'invention peut contenir également des adjuvants habituels dans le domaine cosmétique, tels que les actifs, les conservateurs, les antioxydants, les agents complexants, les solvants, les parfums, les charges, les bactéricides, les absorbeurs d'odeur, les matières colorantes (colorants et pigments) et encore les vésicules lipidiques. Les quantités de ces différents adjuvants sont celles classiquement utilisées dans le domaine considéré, et par exemple de 0,01 à 20 % du poids total de la composition. Ces adjuvants, selon leur nature, peuvent être introduits dans la phase grasse, dans la phase aqueuse et/ou dans les vésicules lipidiques.

Ces adjuvants ainsi que leurs concentrations doivent être tels qu'ils ne modifient pas la propriété recherchée pour la composition de l'invention.

Comme exemples d'actifs utilisables dans la composition selon l'invention, on peut citer par exemple la vitamine B3, encore appelée vitamine PP, sous ses différentes formes, notamment la niacinamide, l'acide nicotinique ou niacine, l'alcool nicotinyle, l'acide nicotinurique et l'acide niconityl hydroxamique, et ses dérivés, notamment les esters de l'acide nicotinique, tels que le nicotinate de tocophérol ; l'adénosine et ses dérivés ; la Vitamine C et ses dérivés, notamment ses esters ; les α-hydroxy-acides notamment les acides dérivés de fruits, comme les acides glycolique, lactique, malique, citrique, tartrique, mandélique, leurs dérivés et leurs mélanges ; les β-hydroxy-acides comme l'acide salicylique et ses dérivés tels que l'acide n-octanoyl-5-salicylique ou l'acide n-dodécanoyl-5 salicylique ; les oligo-éléments comme le cuivre, le zinc, le sélénium, le fer, le magnésium, le manganèse ; les extraits d'algues ; les enzymes ; les coenzymes tels que l'ubiquinone ou coenzyme Q10 qui appartient à la famille des benzoquinones à chaîne alkylénée, le coenzyme R qui est la biotine (ou vitamine H) ; et tout actif approprié pour le but final de la composition, et leurs mélanges.

La composition de l'invention présente l'avantage de pouvoir être exempte d'électrolyte tout en étant bien stable. Ainsi, de manière avantageuse, la composition de l'invention est exempte d'électrolytes tels que le sulfate de magnésium.

La composition cosmétique selon l'invention trouve son application dans un grand nombre de traitements cosmétiques des matières kératiniques, et plus spécialement de la peau, en particulier pour le traitement de la peau, notamment pour hydrater la peau, pour atténuer les irrégularités visibles ou tactiles de la surface de la peau, en particulier pour camoufler les imperfections de la peau et notamment atténuer les rides, les ridules et les taches cutanées, et pour unifier le teint de la peau. Grâce à son agrément et à ses effets, la composition de l'invention est particulièrement appropriée pour le traitement des peaux présentant des imperfections et également des peaux déshydratées ou sensibles.

Des tests sur panel ont montré de bons effets de correction des défauts du teint et d'atténuation des rides, des effets éclaircissants, ainsi que des effets d'unification du teint. En outre, la texture a été jugée fraîche et très agréable à utiliser.

Aussi, la présente invention a pour objet l'utilisation cosmétique de la composition telle que définie ci-dessus, pour le soin de la peau, notamment de la peau deshydratée ou comportant des imperfections.

La présente invention a encore pour objet un procédé cosmétique pour atténuer les irrégularités visibles ou tactiles de la surface de la peau, en particulier pour atténuer les rides et ridules et/ou les taches cutanées et/ou matifier la peau et/ou unifier le teint, comprenant l'application topique sur la peau de la composition telle que définie ci-dessus.

Les exemples ci-après de compositions selon l'invention sont donnés à titre d'illustration et sans caractère limitatif. Les composés sont indiqués en nom chimique ou en nom INCl. Les quantités y sont données en % en poids de matière première, sauf mention contraire.

### Exemples 1 et 1' : crème hydratante éclaircissante

On a réalisé les compositions suivantes : l'exemple 1 selon l'invention comprend plus de 60% d'eau et l'exemple 1' hors invention ne comprend que 55% d'eau.

| | Exemple 1 | Exemple 1' |
|---|---|---|
| *A. Phase aqueuse* | | |
| - Glycérine | 5% | 5% |
| - Conservateur | 0,4% | 0,4% |
| - Polyacrylate de sodium réticulé (Cosmedia SP) | 1 % | 1 % |
| - Eau | 73,84% | 55% |
| *B. Phase huileuse* | | |
| - Cyclohexaméthicone | | |
| - Isohexadécane | 5% | 14,42% |
| - Cire de polyméthylène | 5% | 14,42% |
| - KSG-210 ( Dimethicone /PEG-10/15 crosspolymer) | 2% | 2% |
| (à 27 % en matière active dans dimethicone) (soit 1,08 % d'élastomère de silicone) | 4% | 4% |
| - Distéaryl dimethyl ammonium (Bentone 38 VC) | | |
| - Carbonate de propylène (aide au gonflement de la bentone) | 0,6% | 0,6% |
| | 0,16% | 0,16% |
| *C. Charges* Methylsilanol/silicate crosspolymer (NLK 506) | | |
| | 3% | 3% |

Mode opératoire : on a préparé la phase A par dispersion des constituants, dont le gélifiant Cosmédia, dans l'eau sous agitation avec une turbine Rayneri à 85°C. On a ensuite homogénéisé la phase huileuse à 70°C, puis on a émulsionné en incorporant la phase aqueuse dans la phase huileuse sous forte agitation.

Pour la composition 1, on a obtenu une crème blanche et épaisse. Lors de l'application sur la peau, cette crème était fondante et elle a donné une grande fraîcheur et un grand agrément sensoriel.
Après application, la peau était douce, fraîche et bien hydratée, et les imperfections ont été masquées.

Les propriétés sensorielles (fraîcheur, matité et collant) des deux compositions ont été testées sur un panel de 7 experts. Chaque propriété a été notée sur une échelle de 1 à 5 (1 représentant la borne négative et 5 la borne positive). Les résultats suivants ont été obtenus :

| | Exemple 1 (invention) Note moyenne | Exemple 1' (hors invention) Note moyenne |
|---|---|---|
| Fraîcheur | 3,14 | 2,14 |
| Matité | 3,71 | 1,57 |
| Collant | 1 | 1,29 |

La composition de l'exemple 1 selon l'invention est plus fraîche à l'application que la composition selon l'exemple 1' comparatif, elle est plus mate (significativement moins brillante) et moins collante sur la peau.

## Revendications

1. Composition pour application topique sous forme d'émulsion eau-dans-huile, comprenant une phase aqueuse dispersée dans une phase huileuse, et contenant (1) au moins un émulsionnant siliconé, (2) au moins un polymère hydrophile choisi parmi les polymères acryliques réticulés et les polymères d'acide 2-acrylamido 2-méthylpropane sulfonique, et (3) au moins une charge à base de silicium, la quantité de phase aqueuse étant d'au moins 60 % en poids par rapport au poids total de la composition.

2. Composition selon la revendication 1, **caractérisée en ce que** l'émulsionnant siliconé est choisi parmi les élastomères de silicone émulsionnants, les alkyldiméthicone copolyols et les diméthicone copolyols.

3. Composition selon la revendication 2, **caractérisée en ce que** l'élastomère de silicone émulsionnant est choisi parmi les élastomères de silicone comportant au moins une chaîne oxyalkylénée et/ou une chaîne glycérolée.

4. Composition selon la revendication précédente, **caractérisée en ce que** l'élastomère de silicone comportant au moins une chaîne oxyalkylénée est un élastomère de silicone comportant au moins une chaîne oxyéthylénée.

5. Composition selon la revendication 2, **caractérisée en ce que** l'alkyldimethicone copolyol est choisi parmi ceux comportant un groupe alkyle en C10-C22.

6. Composition selon l'une quelconque des revendications précédentes, **caractérisée en ce que** l'émulsionnant siliconé est présent en une quantité allant de 0,1 à 10 % en poids par rapport au poids total de la composition.

7. Composition selon l'une quelconque des revendications précédentes, **caractérisée en ce que** le polymère acrylique réticulé est choisi parmi le polyacrylate de sodium et les copolymères d'acide acrylique et d'acrylate ou methacrylate d'alkyle en C10-C30.

8. Composition selon l'une quelconque des revendications précédentes, **caractérisée en ce que** le polymère d'acide 2-acrylamido 2-méthylpropane sulfonique est un homopolymère comprenant, distribués de façon aléatoire :
a) de 90 à 99,9% en poids de motifs de formule générale (I) suivante : 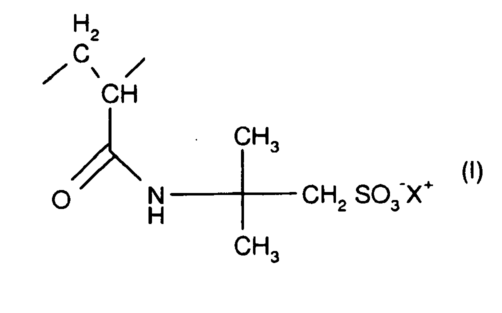 dans laquelle X⁺ désigne un proton, un cation de métal alcalin, un cation alcalino-terreux ou l'ion ammonium, au plus 10% mol des cations X⁺ pouvant être des protons H⁺;
b) de 0,01 à 10% en poids de motifs réticulants provenant d'au moins un monomère ayant au moins deux double-liaisons oléfiniques ; les proportions en poids étant définis par rapport au poids total du polymère.

9. Composition selon l'une quelconque des revendications précédentes, **caractérisée en ce que** le polymère d'acide 2-acrylamido 2-méthylpropane sulfonique est un copolymère choisi parmi :
- les copolymères anioniques réticulés d'acrylamide ou méthylacrylamide et d'acide 2-acrylamido-2-méthylpropane sulfonique,
- les copolymères d'acide(méth)acrylique ou de (méth)acrylate et d'acide 2-acrylamido- 2-méthylpropane sulfonique,
- les copolymères d'acide 2-acrylamido-2-méthylpropane sulfonique et de vinylpyrrolidone ou de vinylformamide
- les copolymères comportant :
- 80 à 99 % en moles de motifs acide 2-acrylamido 2-méthylpropane sulfonique de formule (I) telle que définie dans la revendication précédente, et
- 1 à 20% en moles de motifs hydrophobes de formule (II) suivante : 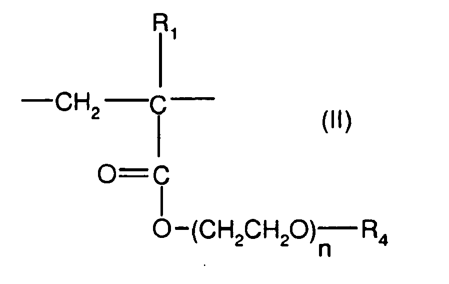 dans laquelle n désigne un nombre entier variant de 3 à 100, de préférence de 3 à 50 et plus préférentiellement de 7 à 25 ; R₁ est l'hydrogène ou un radical méthyle, et R₄ désigne un radical alkyle linéaire ou ramifié comportant de 6 à 22 atomes de carbone, de préférence de 10 à 22 atomes de carbone et mieux de 14 à 22 atomes de carbone.

10. Composition selon l'une quelconque des revendications précédentes, **caractérisée en ce que** la quantité de polymère(s) hydrophile(s) va de 0,1 à 5 % en poids par rapport au poids total de la composition.

11. Composition selon l'une quelconque des revendications précédentes, **caractérisée en ce que** la charge à base de silicium est choisie parmi les silices ayant un diamètre allant de 10 nm à 29 microns et les particules concaves ou annulaires de matériau siliconé.

12. Composition selon la revendication précédente, **caractérisée en ce que** les silices sont des silices poreuses ayant une surface spécifique supérieure à 70m²/g.

13. Composition selon la revendication précédente, **caractérisée en ce que** les particules concaves de matériau siliconé ont un diamètre moyen inférieur ou égal à 10 µm.

14. Composition selon l'une quelconque des revendications 11 à 13, **caractérisée en ce que** les particules concaves de matériau siliconé sont sous forme de portions de sphères creuses ayant une section transversale en forme de fer à cheval ou d'arceau.

15. Composition selon l'une quelconque des revendications 11 à 14, **caractérisée en ce que** le matériau siliconé est un polysiloxane réticulé de structure tridimensionnelle comprenant, ou constitué de, des motifs de formule (I) : SiO₂ et de formule (II) : R¹SiO_{1,5}
dans lesquelles R¹ désigne un groupe organique ayant un atome de carbone directement relié à l'atome de silicium.

16. Composition selon la revendication précédente, **caractérisée en ce que** R¹ est un groupe alkyle en C₁-C₄ ou un groupe phényle.

17. Composition selon la revendication 15, **caractérisée en ce que** R¹ est choisi parmi les groupe époxy, (méth)acryloxy, alkényle, mercaptoalkyle, aminoalkyle, halogénoalkyle, glycéroxy, uréido, cyano.

18. Composition selon l'une quelconque des revendications 15 à 17, **caractérisée en ce que** le matériau siliconé comprend les motifs (I) et (II) selon un rapport molaire motif (I) / motif (II) allant de 30/70 à 50/50, de préférence allant de 35/65 à 45/55.

19. Composition selon l'une quelconque des revendications 11 à 18, **caractérisée en ce que** les particules concaves sont formées d'un petit arc interne (11), d'un grand arc externe (21) et de segments (31) qui relient les extrémités des arcs respectifs, la largeur (W1) entre les deux extrémités du petit arc interne (11) allant de 0,01 à 8 µm, de préférence de 0,02 à 6 µm en moyenne, la largeur (W2) entre les deux extrémités du grand arc externe (21) allant de 0,05 à 10 µm, de préférence de 0,06 à 8 µm en moyenne et la hauteur (H) du grand arc externe (21) allant de 0,015 à 8 µm, de préférence de 0,03 à 6 µm en moyenne.

20. Composition selon la revendication 11, **caractérisée en ce que** les particules de forme annulaire présentent, un diamètre moyen extérieur de 0,05 à 15 µm et un diamètre moyen intérieur de 0,01 à 10 µm; la différence entre le diamètre moyen extérieur et le diamètre moyen intérieur étant de 0,04 à 5 µm.

21. Composition selon la revendication précédente, présentent un réseau polysiloxane comprenant des unités siloxane de formule (1), (2) (3) (4) (5) et (6)
SiO_{4/2} (1)
Si(OH)_{3/2} (2)
R¹SiO_{3/2} (3)
R²SiO_{3/2} (4)
R³ (SiOH)_{2/2} (5)
R⁴(SiOH)_{2/2} (6)
dans lesquelles
- R¹ et R³ désignent des groupes hydrocarbonés non réactifs, notamment des groupes alkyle, cycloalkyle, aryle, alkylaryle ou aralkyle, de préférence les groupes alkyles en C₁-C₃ ;
- R² et R⁴ désignent chacun un groupe hydrocarboné choisi parmi les groupes acryloxy, methacryloxy, vinyle ou mercapto ;
- le rapport molaire unités siloxane de formule (1) / unités siloxane de formule (2), (3), (4), (5) et (6) étant de 20/80 à 50/50 ;
- le rapport molaire unités siloxane de formule (2), (3) et (4) / unités siloxane de formule (5) et (6) étant de 50/50 à 75/25;
- le rapport molaire unités siloxane de formule (3) et (5) / unités siloxane de formule (4) et (6) étant de 20/80 à 60/40.

22. Composition selon l'une quelconque des revendications précédentes, **caractérisée en ce que** la quantité de charge(s) à base de silicium va de 0,1 à 10 % en poids par rapport au poids total de la composition.

23. Composition selon l'une quelconque des revendications précédentes, **caractérisée en ce que** la phase huileuse contient en outre un épaississant.

24. Composition selon l'une quelconque des revendications précédentes, **caractérisée en ce que** la phase aqueuse est présente en une quantité allant de 60 à 95 % par rapport au poids total de la composition.

25. Composition selon l'une quelconque des revendications précédentes, **caractérisée en ce qu'**elle contient au moins 30 % d'eau en poids, et de préférence au moins 40 % d'eau en poids par rapport au poids total de la composition.

26. Utilisation cosmétique de la composition selon l'une quelconque des revendications 1 à 25, pour le soin de la peau.

27. Procédé cosmétique pour atténuer les irrégularités visibles ou tactiles de la surface de la peau, comprenant l'application topique sur la peau de la composition selon l'une quelconque des revendications 1 à 25.

## Claims

1. Composition for topical application in the form of a water-in-oil emulsion comprising an aqueous phase dispersed in an only phase, and containing (1) at least one silicone emulsifier, (2) at least one hydrophilic polymer chosen from crosslinked acrylic polymers and 2-acrylamido-2-methylpropanesulfonic acid polymer, and (3) at least one silicon-based filler, the amount of aqueous phase being at least 60% by weight relative to the total weight of the composition.

2. Composition according to Claim 1, **characterized in that** the silicone emulsifies is chosen from emulsifying silicone elastomer, alkyldimethicone copolyols and dimethicone copolyols.

3. Composition according to Claim 2, **characterized in that** the emulsifying silicone elastomer is chosen from silicone elastomer comprising at least one oxyalkylenated chain and/or one glycerolated chain.

4. Composition according to the receding claim, **characterized in that** the silicone elastomer comprising at least one oxyalkylenated chain is a silicone elastomer comprising at least one oxy-ethylenated chain.

5. Composition according to Claim 2, **characterized in that** the alkyldimethicone copolyol is chosen from those comprising a C10-C22 alkyl group.

6. Composition, according to any one of the receding claims, **characterized in that** the silicone emulsifier is present in an amount ranging from 0.1% to 10% by weight relative to the total weight of the composition.

7. Composition according to any one of the preceding claims, **characterized in that** the crosslinked acrylic polymer is chosen from sodium polyacrylate and copolymers of acrylic acid and of C10-C30 alkyl acrylate or methacrylate.

8. Composition according to any one of the receding claims, **characterized in that** the 2-acrylamido-2-methylpropanesulfonic acid polymer is a homopolymer comprising, randomly distributed:
a) from 90% to 99.9% by weight of units of general formula (I) below: 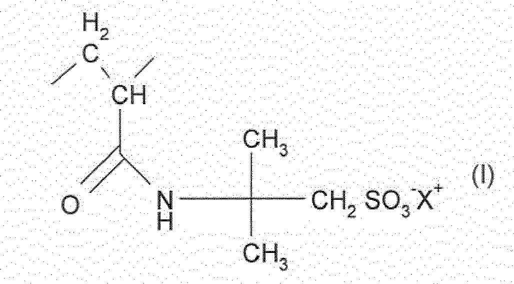 in which X⁺ denotes a photon, an alkali metal cation, an alkaline-earth metal cation or the ammonium ion, not more than 10 mol% of the cations X⁺ possible being protons H⁺;
b) from 0.01% to 10% by weight of crosslinking units derived from at least one monomer containing at least two olefinic doubly bonds; the weight proportion being defied relative to the total weight of the polymer.

9. Composition according to any one of the preceding claims, **characterized in that** the 2-acrylamido-2-methylpropanesulfonic acid polymer is a copolymer chosen from:
- crosslinked anionic copolymers of acrylamide or methacrylamide and of 2-acrylamido-2-methylpropanesulfonic acid,
- copolymers of (meth)acrylic acid or of (meth)acrylate and of 2-acrylamido-2-methylpropanesulfonic acid,
- copolymer of 2-acrylamido-2-methylpropanesulfonic acid and of vinylpyrrolidone or vinylformamide,
- copolymers comprising:
- 80 mol% to 99 mol% of 2-acrylamido-2-methylpropanesulfonic acid units of formula (I) as defined in the preceding claim,
and
- 1 mole% to 20 mol% of hydrophobic units of formula (II) below: 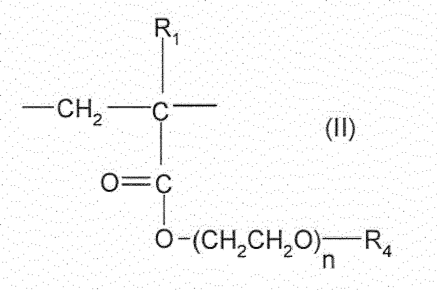 in which n denotes an integer ranging from 3 to 100, preferable from 3 to 50 and more preferentially from 7 to 25; R₁ is hydrogen or a methyl radical, and R₄ denotes a linear or branched alkyl radical containing from 6 to 22 carbon atoms, preferable from 10 to 22 carbon atoms and better still from 14 to 22 carbon atoms.

10. Composition according to any one of the preceding claims, **characterized in that** the amount of hydrophilic polymer(s) ranges from 0.1% to 5% by weight relative to the total weight of the composition.

11. Composition according to any one of the preceding claims, **characterized in that** the silicon-based filler is chosen from silicas with a diameter ranging from 10 nm to 29 microns and concave or circular particles of silicone material.

12. Composition according to the preceding claim **characterized in that** the silicas are porous silicas with a specific surface area of greater than 70 m²/g.

13. Composition according to the receding claim, **characterized in that** the concave particles of silicone material have a mean diameter of less than or equal to 10 µm.

14. Composition according to any one of Claims 11 to 13, **characterized in that** the concave particles of silicon material are in the form of hollow sphere portions with a horseshoe-shaped or bow-shaped cross section.

15. Composition according to any one of Claims 11 to 14, **characterized in that** the silicone material is a crosslinked polysiloxane of three-dimensional structure comprising, or constituted of, units of formula (I): SiO₂, and of formula (II): R¹SiO_{1.5} in which R¹ denotes an organic group containing a carbon atom directly bonded to the silicone atom.

16. Composition according to the preceding claim, **characterized in that** R¹ is a C₁-C₄ alkyl group or a phenol group.

17. Composition according to Claim 15, **characterized in that** R¹ is chosen from epoxy, (meth)acryloxy, alkenyl, mercaptoalkyl, aminoalkyl, haloalkyl, glyceroxy, ureido and cyano groups.

18. Composition according to any one of Claims 15 to 17, **characterized in that** the silicone material comprises the units (I) and (II) in a unit (I)/unit (II) mole ratio ranging from 30/70 to 5//50 and preferable ranging from 35/65 to 45/55.

19. Composition according to any one of Claims 11 to 18, **characterized in that** the concave particles are formed of a small infer arc (11), a large outer arc (21) and segments (31) that connect the ends of the respective arcs, the width (W1) between the two ends of the small inner arc (11) ranging from 0.01 to 8 µm and preferable from 0.02 to 6 µm on average, the width (W2) between the two ends of the large outer arc (21) ranging from 0.05 to 10 µm and preferable from 0.06 to 8 µm on average and the height (H) of the large outer arc (21) ranging from 0.015 to 8 µm and preferably from 0.03 to 6 µm on average.

20. Composition according to Claim 11, **characterized in that** the ring-shaped particles have a mean outside diameter of from 0.05 to 15 µm and a mean inside diameter of from 0.01 to 10 µm; the difference between the mean outside diameter and the mean inside diameter being from 0.04 to 5 µm.

21. Composition according to the preceding claim, having a polysiloxane network comprising siloxane units of formulae (1), (2), (3), (4), (5) and (6):
SiO_{4/2} (1)
Si(OH)_{3/2} (2)
R¹SiO_{3/2} (3)
R²SiO_{3/2} (4)
R³ (SiOH)_{2/2} (5)
R⁴ (SiOH)_{2/2} (6)
in which
- R¹ and R₃ denote unreactive hydrocarbon-based groups, specially alkyl, cycloalkyl, aryl, alkylaryl or aralkyl groups, preferably C₃-C₃ alkyl groups;
- R⁴ and R⁴ each denote a hydrocarbon-based group chosen from acryloxy, methacryloxy, vinyl and mercapto groups;
- the mole ratio or siloxane units of formula (1)/siloxane units of formulae (2), (3), (4), (5) and (6) being from 20/80 to 50/50;
- the mole ratio of siloxane units of formulae (2), (3) and (4)/siloxane units of formulae (5) and (6) being from 50/50 to 75/25;
- the mole ratio of siloxane units of formulae (3) and (5)/siloxane units of formulae (4) and (6) being from 20/80 to 60/40.

22. Composition according to any one of the preceding claims, **characterized in that** the amount of silicon-based filler(s) ranges from 0.1% to 10% by weight relative to the total weight of the composition.

23. Composition according to any one of the preceding claims, **characterized in that** the oily phase also contains a thickener.

24. Composition according to any one of the preceding claims, **characterized in that** the aqueous phase is prevent in an amount ranging from 60% to 95% relative to the total weight of the composition.

25. Composition according to any one of the preceding Claims, **characterized in that** it contains at least 30% by weight of water and preferably at least 40% by weight of water relative to the total weight of the composition.

26. Cosmetic use of the composition according to any one of Claims 1 to 25, for caring for the skin.

27. Cosmetic process for attenuating the visible or tactile irregularities of the surface of the skin, comprising the topical application to the skin of the composition according to any one of Claims 1 to 25

## Patentansprüche

1. Zusammensetzung für die topische Abwendung in Form einer Wasser-in-Öl-Emulsion, die eine in einer Ölphase dispergierte Wasserphase umfaßt und die (1) mindestens einen Silikonemulgator, (2) mindestens ein hydrophiles Polymer, ausgewählt aus der Reihe der vernetzten Acrylpolymere und der 2-Acrylamido-2-methylpropansulfonsäure-Polymere, sowie (3) mindestens einen Füller auf Siliciumbasis enthält, wobei die Menge an wässriger Phase mindestens 60 Gew.-% in Bezug auf das Gesamtgewicht der Zusammensetzung beträgt.

2. Zusammensetzung nach Anspruch 1, dadurch gekenntzeichnet, dass der Silikonemulgator aus der Reihe der emulgierenden Silikonelastomere, Alkyldimethicon-Copolyole und Dimethiconcopolyole stammt.

3. Zusammensetzung nach Anspruch 2, **dadurch gekennzeichnet, dass** das emulgierende Silikonelastomer aus der Reihe der Silikonelastomere, die mindestens eine Oxyalkylenkette und/oder eine Glycerolkette umfassen, stammt.

4. Zusammensetzung nach dem vorhergehenden Anspruch, **dadurch gekennzeichnet, dass** es sich bei dem Silikonelastomer, das mindestens eine Oxyalkylenkette umfaßt, um ein Silikonelastomer, das mindestens eine Oxyethylenkette umfaßt, handelt.

5. Zusammensetzung nach Anspruch 2, dadurch gekenntzeichnet, dass das Alkyldimethicon-Copolyol aus der Reihe derjenigen, die eine C10-C22-Alkylgruppe umfassen, stammt.

6. Zusammensetzung nach einem der vorhergehenden Ansprüche, **dadurch gekennzeichnet**, das der Silikonemulgator in einer Menge von 0,1 bis 10 Gew.-% in Bezug auf das Gesamtgewicht der Zusammensetzung vorliegt.

7. Zusammensetzung nach einem der vorhergehender Ansprüche, **dadurch gekennzeichnet**, das das vernetzte Acrylpolymer aus der Reihe Natriumpolyacrylat und Acrylsäure-C10-C30-alkylacrylat- oder -methacrylat-Copolymere stammt.

8. Zusammensetzung nach einem der vorhergehenden Ansprüche, dadurch gekenntzeichnet, dass es sich bei dem 2-Acrylamido-2-methylplopansulfonsäure-Polymer um ein Homopolymer handelt, das zufallsmäßig verteilt Folgendes umfasst:
a) 90 bis 99,9 Gew.-% Einheiten der folgenden allgemeinem Formel (I): 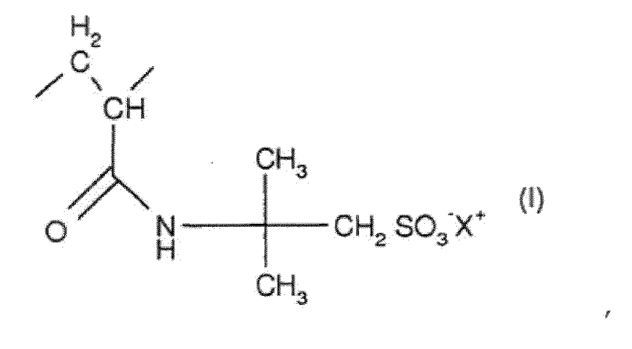 worin X⁺ ein Proton, ein Alkalimetallkation, ein Erdalkalimetallkation oder das Ammoniumion bedeutet, wobei maximal 10 mol-% der Kationen X⁺ Protonen H⁺ sein können;
b) 0,01 bis 10 Gew.-%. vernetzende Einheiten, die von mindestens einem Monomer mit mindestens zwei olefinischen Doppelbindungen stammen; wobei die Gewichtsverhältnisse in Bezug auf das Gesamtgewicht des Polymers angegeben sand.

9. Zusammensetzung nach einem der vorhergehender Ansprüche, **dadurch gekennzeichnet, dass** es sich bei dem 2-Acrylamido-2-methylpropansulfonsäure-Polymer um ein Copolymer handelt, das aus Folgendem ausgewählt ist:
- vernetzte anionische Copolymere von Acrylamid oder ethylacrylamid und 2-acrylamido-2-methylpropansulfonsäure,
- Copolymere der (Meth)acrylsäure oder des (Meth)acrylats und der 2-Acrylamido-2-methylpropansulfonsäure,
- Copolymere der 2-Acrylamido-2-methylpropansulfonsäure und des Vinylpyrrolidons oder vinylformamids,
- wobei die Copolymere Folgender umfassen:
- 80 bis 99 mol-% 2-acrylamido-2-methylpropansulfonsäure - Einheiten der Formel (I) wie im vorhergehenden Anspruch definiert, und
- 1 bis 20 mol-% hydrophobe Einheiten der folgenden Formel (II): 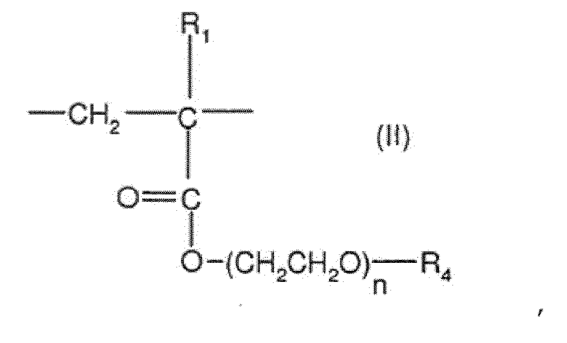 worin n eine ganze Zahl von 3 bis 100, vorzugsweise von 3 bis 50 und stärker bevorzugt von 7 bis 25 bedeutet; R₁ Wasserstoff oder einen Methylrest bedeutet, und R₄ einen geradkettigen oder verzweigten Alkylrest mit 6 bis 22 Kohlenstoffatomen, vorzugsweise mit 10 bis 22 Kohlenstoffatomen und besser mit 14 bis 22 Kohlenstoffatomen, aufweist.

10. Zusammensetzung nach einem der vorhergehenden Ansprüche, **dadurch gekennzeichnet, dass** die Menge an hydrophilen Polymer bzw. hydrophilen Polymeren von 0,1 bis 5 Gew.-% in Bezug auf das Gesamtgewicht der Zusammensetzung ausmacht.

11. Zusammensetzung nach einem der vorhergehenden Ansprüche, **dadurch gekennzeichnet, dass** der Füller auf Siliciumbasis aus der Reihe der Siliciumdioxide mit einem Durchmesser von 10 nm bis 29 Mikrometer und der konkaven oder ringförmigem Partikeln aus Silikonmaterial stammt.

12. Zusammensetzung nach dem vorhergehenden Anspruch, **dadurch gekennzeichnet, dass** es sich bei den Siliciumdioxiden um poröse Siliciumdioxids mit einer spezifische Oberfläche von mehr als 70 m²/g handelt.

13. Zusammensetzung nach dem vorhergehenden Anspruch, **dadurch gekennzeichnet, dass** die konkaven Partikel aus Silikonmaterial einen mittleren Durchmesser von 10 µm oder darunter aufweisen.

14. Zusammensetzung nach einem der Ansprüche 11 bis 13, dadurch gekenntzeichnet, dass die konkaven Partikel aus Silikonmaterial in Form von Hohlkugelteilen mit einen hufeisenförmigen oder bogenförmigen Querschnitt vorliegen.

15. Zusammensetzung nach einem der Ansprüche 11 bis 14, dadurch gekenntzeichnet, dass es sich bei dem Silikonmaterial um ein vernetzte Polysiloxan mit dreidimensionaler Struktur handelt, das Einheiten der Formel (I): SiO₂ und der Formel (II): R¹SiO_{1,5},
worin R¹ eine organische Gruppe mit einem direkt an das Siliciumatom gebundenen Kohlenstoffatom bedeutet, umfasst bzw. aus diesen Einheiten besteht.

16. Zusammensetzung nach dem vorhergehenden Anspruch, **dadurch gekennzeichnet, dass** R¹ eine C₁-C₄-Alkylgruppe oder eine Phenylgruppe bedeutet.

17. Zusammensetzung nach Anspruch 15, **dadurch gekennzeichnet, dass** R¹ aus den Epoxy-, (Meth)acryloxy-, Alkenyl-, Mercaptoalkyl-, aminoalkyl-, Halogenalkyl-, Glyceroxy-, Ureido-, Cyanogruppen stammt.

18. Zusammensetzung nach einem der Ansprüche 15 bis 17, **dadurch gekennzeichnet, dass** das Silikonmaterial die Einheiten (I) und (II) in einem molarem Verhältnis von Einheit (I)/Einheit (II) von 30/70 bis 50/50, vorzugsweise von 35/65 bis 45/55, umfasst.

19. Zuaammensetzung nach einem der Anspruche 11 bis 18, **dadurch gekennzeichnet**, das die konkaven Partikel von einem kleinen inneren Bogen (11), einem großen äußeren Bogens (21) und Segmenten (31), die die Enden der jeweiligen Bögen verbinden, gebildet sind, wobei die Weite (W1) zwischen den beiden Enden des kleinen inneren Bogens (11) 0,01 bis 8 µm, vorzugsweise im Mittel 0,02 bis 6 µm, beträgt, die Weite (W2) zwischen den beiden Enden des großen äußeren Bogens (21) 0,05 bis 10 µm, vorzugsweise im Mitten 0,06 bis 8 µm, beträgt und die Höhe (H) des großen äußeren Bogens (21) 0,015 bis 8 µm, vorzugsweise im Mittel von 0,03 bis 6 µm, beträgt.

20. Zusammensetzung nach Anspruch 11, dadurch gekenntzeichnet, dass die Partikel in Ringform einen mittleren äußeren Durchmesser von 0,05 bis 15 µm und einen mittleren inneren Durchmesser von 0,01 bis 10 µm ausweisen, wobei die Differenz zwischen dem mittleren äußeren Durchmesser und dem mittleren inneren Durchmesser 0,04 bis 5 µm beträgt.

21. Zusammensetzung nach dem vorhergehenden Anspruch, die ein Polysiloxannetz mit Siloxaneinheiten der Formel (1), (2), (3), (4), (5) und (6) aufweist:
SiO_{4/2} (1)
Si-(OH)_{3/2} (2)
R¹SiO_{3/2} (3)
R²SiO_{3/2} (4)
R³ (SiOH)_{2/2} (5)
R⁴ (SiOH)_{2/2} (6),
worin
- R¹ und R³ nicht reaktionsfähige Kohlenwasserstoffgruppen, insbesondere Alkyl-, Cycloalkyl-, acryl-, Alkylaryl- oder Aralkylgruppen, vorzugsweise C₁-C₃-Alkylgruppen, bedeuten;
- R² und R⁴ jeweils eine Kohlenwasserstoffgruppe aus den Gruppen Acryloxy, Methacryloxy, Vinyl oder Mercapto bedeuten;
- das Molverhältnis von Siloxaneinheiten der Formel (1)/Siloxaneinheiten der Formel (2), (3), (4), (5) und (6) 20/80 bis 50/50 beträgt;
- das Molverhältnis von Siloxaneinheiten der Formel (2), (3) und (4)/Siloxaneinheiten der Formel (5) und (6) 50/50 bis 75/25 beträgt;
- das Molverhältnis von Siloxaneinheiten der Formel (3) und (5) Siloxaneinheiten der Formel (4) und (6) 20/80 bis 60/40 beträgt.

22. Zusammensetzung nach einem der vorhergehenden Ansprüche, **dadurch gekennzeichnete dass** die Menge an Füller (n) auf Siliciumbasis von 0,1 bis 10 Gew.-% in Bezug auf das Gesamtgewicht der zusammensetzung ausmacht.

23. Zusammensetzung nach einem der vorhergehender Ansprüche, dadurch gekenntzeichnet, dass die Ölphase weiterhin ein Verdickungsmittel enthält.

24. Zusammensetzung nach einem der vorhergehenden Ansprüche, **dadurch gekennzeichnet**, das die Wasserphase in einer Menge von 60 bis 95% in Bezug auf das Gesamtgewicht der Zusammensetzung vorlegt.

25. Zusammensetzung nach einem der vorhergehenden Ansprüche, **dadurch gekennzeichnet**, das sie mindestens 30 Ges.-% wasser, vorzugsweise mindestens 40 Gew.-% Wasser, in Bezug auf das Gesamtgewicht der Zuaammensetzung enthält.

26. Kosmetische Verwerdung der Zusammensetzung nach einem der Ansprüche 1 bis 25 für die Hautpflege.

27. Kosmetischer Verfahren zum Mindern der sichtbaren oder fühlbaren Unregelmäßigkeiten der Hauptoberfläche, bei dem man die Zusammensetzung nach einem der Ansprüche 1 bis 25 topisch auf die Haut aufträgt.
